# Europäisches Patentamt
# European Patent Office
# Office européen des brevets

(19)

(11) Veröffentlichungsnummer: **0 001 426**

|B1

(12) **EUROPÄISCHE PATENTSCHRIFT**

(21) Anmeldenummer: **78100982.4**

(22) Anmeldetag: **25.09.78**

(51) Int. Cl.³: **C 07 C 17/24,**
**C 07 C 17/38,**
**C 07 C 21/04,**
**B 01 J 23/94**

(54) **Verfahren zum Trocknen von kupferhaltigem Katalysatorabfall**

(30) Priorität: **07.10.77 DE 2745078**

(43) Veröffentlichungstag der Anmeldung:
**18.04.79 Patentblatt 79/08**

(45) Bekanntmachung des Hinweises auf die Patenterteilung:
**17.09.80 Patentblatt 80/19**

(84) Benannte Vertragsstaaten:
**BE DE FR GB NL**

(56) Entgegenhaltungen:
**DE - A - 2 200 780**
**DE - A - 2 532 472**
**FR - A - 2 281 777**

(73) Patentinhaber: **BAYER AG**
**Zentralbereich Patente, Marken und Lizenzen**
**D - 5090 Leverkusen 1, Bayerwerk (DE)**

(72) Erfinder: **Beilstein, Günter, Dr.**
**Iltisweg 64**
**D - 4041 Dormagen 11 (DE)**
**Casper, Clemens, Dr.**
**Giesenweg 76**
**D - 4150 Krefeld (DE)**
**Grenner, Dieter, Dr.**
**Goethestrasse 57c**
**D - 4047 Dormagen (DE)**
**Sajben, Johannes Otto**
**Bethelstrasse 64**
**D - 4150 Krefeld (DE)**

Courier Press, Leamington Spa, England.

Verfahren zum Trocknen von kupferhaltigem Katalysatorabfall

Die Erfindung betrifft ein Verfahren zum Trocknen des bei einer Isomerisierungsreaktion in Form einer Suspension anfallenden, kupferhaltigem Katalysatorabfalls unter Wiedergewinnung von Dichlorbuten.

Bei einer Isomerisierung von 1,4-Dichlorbuten-2 zu 3,4-Dichlorbuten-1 oder umgekehrt werden zur Beschleunigung der Gleichgewichtseinstellung zwischen den beiden Isomeren, von denen jeweils das neu gebildete aus der Reaktionsmischung, z.B. destillativ, entfernt wird, verschiedene Katalysatoren eingesetzt, die zum überwiegenden Teil auf Komplexen von Kupfer mit organischen Komplexbildern beruhen. Als solche kommen z.B. in Frage: Adipinonitril, Benzonitril (GB—PS 1 260 681), quaternäre Ammoniumverbindung (US—PS 203 476 = DT—OS 2 248 668), Pyridin (JA—PS 8 451/68), Benzonitril (JA—PS 8 453/68), Naphthenate, Oleate, Stearate zusammen mit substituierten Harnstoffen (DT—OS 2 212 235). Auch die Anwendung nicht komplexierten Kupfer-I-chlorids ist beschrieben (DT—AS 1 233 385).

Die Isomerisierung läuft endotherm bei durchweg erhöhten Temperaturen (50—150°C) ab. Dabei bilden sich höhersiedende Nebenprodukte, welche sich in der Reaktionsmischung anreichern. Diese Nebenproduktbildung ist auch in einer Reihe von Patentschriften genannt (DT—OS 1 950 971, DT—OS 1 802 385, JA—PS 420/66, BE—PS 788 356, BE—PS 763 117, DT—AS 1 220 847), wobei erwähnt wird, daß bei geringen Verweilzeiten (erreichbar z.B. durch optimale Katalysatoren) die Nebenproduktbildung geringer wird.

Da es bis jetzt nicht gelungen ist, durch geeignete Reaktionsführung die Schwersiederbildung ganz zu unterdrücken, ist es zur Vermeidung eines ständigen Anwachsens der Schwersiederkonzentration notwendig, einen Anteil der Reaktionsmischung auszuschleusen. Dieser Anteil muß vernichtet werden, wobei eine Verbrennung wegen der HCl-Entwicklung aus dem Dichlorbuten sowie der Bildung von kupferhaltigem Staub mit erheblichen Komplikationen bei der Rauchgasreinigung verbunden ist.

In der DT—OS 2 532 472 erfolgt die Aufarbeitung des gelösten Kupferkomplexes unter Zusatz eines unpolaren Lösungsmittels durch Ausfällung und Filtrierung. Dieses Verfahren hat den Nachteil hoher Investitions- und Betriebskosten.

In der DT—OS 1 220 847 werden die höhersiedenden Bestandteile abdestilliert. Die Aufarbeitung ist aufwendig. Auch treten bei Lagerung der mit Kupferverbindung kontaminierten flüssigen Schwersieder Umweltprobleme auf.

Der Erfindung liegt die Aufgabe zugrunde, ein Verfahren zu finden, bei dem das Dichlorbuten möglichst vollständig wiedergewonnen wird und der Katalysatorabfall aus kupferhaltigen Hochsiedern in einer festen Phase anfällt, die zwecks umweltfreundlicher Deponie wenig Neigung zur Zersetzung und Aggression besitzt.

Diese Aufgabe wird erfindungsgemäß dadurch gelöst, daß die Suspension mit 1—15 Gew.-% Feststoffgehalt bei einer Temperatur von 70—150°C in ein stetig gewendelten Rohr, dessen Verhältnis von Rohrdurchmesser zu Krümmungsdurchmesser 0,01:1 bis 0,1:1 beträgt, entspannt wird, wobei mindestens 15 Gew.-% der Suspension verdampft werden, und wobei die restliche Suspension anschließend beim Durchgang durch das Rohr bei einem Druckverlust von 100—2000 mbar mit gleichzeitigem Temperaturrückgang auf 50—120°C getrocknet wird, um danach in einem Abscheider in festen, partikelförmigen Katalysatorabfall und gasförmiges Dichlorbuten getrennt zu werden.

Die mit der Erfindung erzielten Vorteile bestehen insbesondere darin, daß neben der Wiedergewinnung von Dichlorbuten ein fester partikelförmiger Abfallstoff mit geringer Eigenfeuchtigkeit anfällt, der wegen der Kompaktheit, geringer Neigung zur chemischen Veränderung und weitgehender Ablegung der Aggressivität nunmehr umweltfreundlich z.B. in normalen Behältern oder Fässern zu lagern ist.

Für den Fachmann war es daher überraschend, daß der teerähnliche Zustand beim Übergang von der flüssigen zur Trockenphase nicht zu Verstopfungen oder zumindest örtlichen Überhitzungen führte, die eventuell das Rohr zerstören würden und/oder einen inhomogenen Abfallstoff ergäben, dessen Lagerung wegen Zersetzung der Substanz bzw. exothermer Polymerisation problematisch ist.

Auch was nicht voraussehbar, wie sich eine Suspension, bei der die einzelnen Bestandteile sehr unterschiedliche Reibungskoeffizienten haben, insbesondere im Phasenübergang bei einem Dampfstrom verhält.

Das Verfahren erlaubt eine kurze Verweilzeit bei vorzugebender Temperaturführung, so daß es optimal gefahren werden kann. Die Ausbeute ist groß. Das Verfahren mit einem wendelförmigen Rohr ist sehr wirtschaftlich, da das verdampfte Dichlorbuten gleichzeitig als Treibmittel genützt wird und zum Schluß wiedergewonnen wird.

Ein Ausführungsbeispiel der Erfindung ist in der Zeichnung dargestellt und wird im folgenden näher beschrieben.

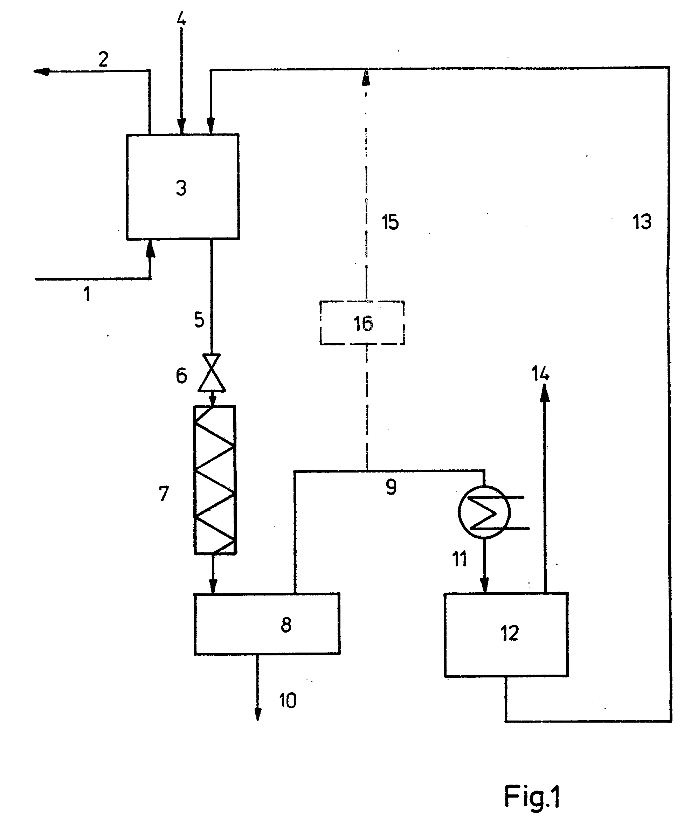

In der Fig. 1 wird 1,4-Dichlorbuten-2 über eine Leitung (1) in einem Isomerisierungsreaktor (3) eingeführt. Gleichzeitig wird ein Katalysator über eine Leitung (4) zugesetzt. In endothermer Reaktion wandelt sich ein Teil des 1,4-Dichlorbuten-2 in 3,4-Dichlorbuten-1 um. Das entstehende Gemisch beider Isomeren wird über Leitung (2) zur Gewinnung des 3,4-Dichlorbutens einer nicht dargestellten Destillationseinrichtung zugeführt.

Ein Teil des Reaktorinhalts wird kontinuierlich oder diskontinuierlich über eine Leitung (5) abgezogen und über ein Entspannungsventil (6) in ein stetig gewendelten Rohr (7) eingedüst und darin so eingedampft, daß in einer nachgeschalteten Abscheidekammer (8) ein festes, partikelförmiges Produkt anfällt, während die Dichlorbutene gasförmig über eine Leitung (9) abgezogen werden. Das Festprodukt wird über eine Leitung (10) ausgetragen. Es verändert sich bei weiterer Lagerung nicht mehr und kann einer Deponie zugeführt werden. Dies geschieht zweckmäßigerweise nach Abfüllung in Fässer oder Säcke aus geeignetem Material.

Die Dichlorbutene werden in einem Kondensator (11) verflüssigt und in einer Vorlage (12) gesammelt, von wo aus sie über eine Leitung (13) wieder in den Isomerisierungsreaktor (3) zurückgepumpt werden können. Dabei sind etwa mitgerissene Feststoffanteile nur in vernachlässigbar geringen Mengen vorhanden, so daß die Funktion des Isomerisierungsreaktors (3) durch die Rückführung nicht beeinträchtigt wird. Über Leitung (14) schließlich ist die Anlage an ein Vakuumsystem (nicht dargestellt) angeschlossen.

Eine -gestrichelt gezeichnete-Variante besteht darin, daß man das gasförmige Dichlorbutengemisch ohne Zwischenkondensation über Leitung (15) und eine Vakuumstation (16) direkt in den Reaktor zurückdrückt.

### Beispiel

Aus einem Isomerisierungsreaktor werden zur Aufrechterhaltung der Konzentrationsverhältnisse von

1,8% Kupferchlorid in komplex gebundener Form
8,1% Hochsieder und Feststoffe
12,1% 3,4-Dichlorbuten-1 und
78,0% 1,4-Dichlorbuten-2

120 kg/h schwersiedender Katalysatorabfall ausgeschleust und einem stetig gewendelten Rohr (7) (Nennweite 25 mm, Krümmungsdurchmesser 650 mm, Länge 18 m) unter Entspannung bei einem Abfall der Temperatur von 128°C auf 97°C zugeführt. Die Eindampfung erfolgt bei einem Druckverlust von 500 mbar so, daß ein fester Rückstand in einer Menge von 18,7 kg/h anfällt, welcher noch adsorptiv gebundenes Dichlorbuten (innere Feuchtigkeit) enthält. Am Ende des gewendelten Rohres (7) beträgt die Austrittstemperatur 80°C und der Druck 70 mbar.

Die über (8) und (9) abziehenden Brüden werden im Kondensator (11) verflüssigt und im Sammler (12) gesammelt. Das Kondensat (101,3 kg/h) enthält neben den Dichlorbutenen als Hauptbestandteilen noch 0,09% Kupferchlorid sowie 0,22% Hochsieder und wird über die Leitung (13) wieder in den Isomerisierungsreaktor (3) eingespeist. Durch die Rückführung wird die Ausbeute an Dichlorbutenen um diese Menge verbessert.

### Patentanspruch

Verfahren zum Trocknen des bei einer Isomerisierungsreaktion in Form einer Suspension anfallenden kupferhaltigen Katalysatorabfalls unter Wiedergewinnung von Dichlorbuten, dadurch gekennzeichnet, daß die Suspension mit 1—15 Gew.-% Feststoffgehalt bei einer Temperatur von 70—150°C in ein stetig gewendelten Rohr, dessen Verhältnis von Rohrdurchmeser zu Krümmungsdurchmesser 0,01:1 bis 0,1:1 beträgt, entspannt wird, wobei mindestens 15 Gew.-% der Suspension verdampft werden; und wobei die restliche Suspension anschließend beim Durchgang durch das Rohr bei einem Druckverlust von 100—2000 mbar mit gleichzeitigem Temperaturrückgang auf 48—120°C getrocknet wird, um danach in einem Abscheider in festen, partikelförmigen Katalysatorabfall und gasförmiges Dichlorbuten getrennt zu werden.

### Revendication

Procédé de séchage des déchets de catalyseur contenant du cuivre que l'on rencontre dans une réaction d'isomérisation sous forme d'une suspension, avec récupération du dichlorobutène, caractérisé en ce qu'on détend la suspension contenant 1—15% en poids de teneur en matière sèche à une température de 70—150°C dans un tube hélicoïdal continu, dont le rapport du diamètre de tube au diamètre de courbure est de 0,01:1 à 0,1:1, en vaporisant au moins 15% en poids de la suspension, en ce que la suspension restante est ensuite séchée lors du passage à travers le tube avec une perte de pression de 100 à 2000 mbars avec retour simultané de la température à 48—120°C, pour ensuite être séparée dans un séparateur en des déchets de catalyseur solides sous forme de particules et en dichlorobutène gazeux.

### Claim

Process for drying the copper-containing catalyst waste products obtained in the form of a suspension during an isomerisation reaction, dichlorobutene being recovered, characterised in that the suspension, containing 1—15% by weight of solids, is let down, at a temperature of 70—150°C, into a tube which is in the form of a continuous spiral and in which the ratio of tube diameter to diameter of curvature is 0.01:1 to 0.1:1, at least 15% by weight of the suspension being evaporated; and the remainder of the suspension is then dried, on passage through the tube, with a pressure loss of 100—2000 mbars and a simultaneous drop in temperature of 48—120°C, and is then separated, in a separator, into solid, granular catalyst waste products and gaseous dichlorobutene.

Fig.1

0 001 426

1